# EUROPÄISCHE PATENTANMELDUNG

(11) **EP 1 103 181 A1**
(43) Veröffentlichungstag der Anmeldung: **30.05.2001**
(21) Anmeldenummer: 99811085.2
(22) Anmeldetag: 25.11.1999
(51) Int. Cl.: A01N 43/90

(54) **Kombination der N-Phenyl-N'-Benzoyl Harnstoff Derivaten und Avermectin für Parasiten Kontrolle.**

(71) Anmelder: Novartis AG, 4058 Basel (CH)
(72) Erfinder: Die Erfindernennung liegt noch nicht vor
(74) Vertreter: Becker, Konrad

(57) **Zusammenfassung**

Es wird eine Wirkstoffkombination zur gleichzeitigen Bekämpfung von Ekto- und Endoparasiten, insbesondere Milben, Zecken und Nematoden, bei Nutz-, Haus- und Schosstieren beschrieben, die aus deen Wirkstoffen
(1) 1-[4-Chlor-3-(3-chlor-5-trifluormethyl-2-pyridyloxy)phenyl]-3-(2,6- difluorbenzoyl)harnstoff und
(2) einer Verbindung der Formel (i) besteht 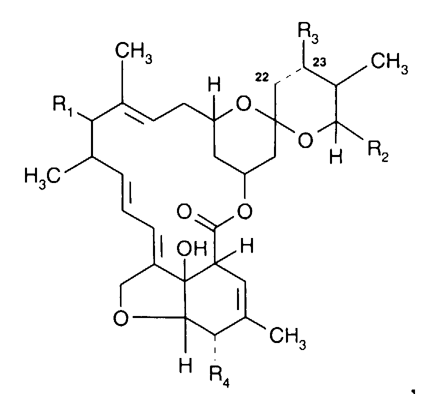 worin R₁ für Wasserstoff oder für einen der Reste 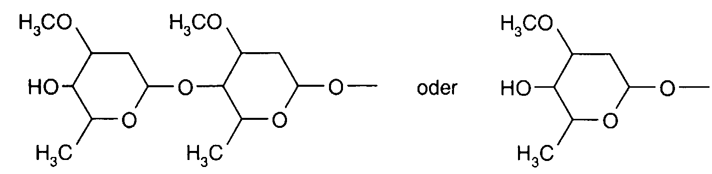 steht; R₂ für -CH(CH₃)-CH₃, -CH(CH₃)-C₂H₅, -C(CH₃)=CH-CH(CH₃)₂ oder Cyclohexyl steht; R₃ für Wasserstoff oder Hydroxy steht, wenn die Bindung zwischen den Atomen 22 und 23 eine Doppelbindung repräsentiert, oder für Wasserstoff oder die Gruppe =N-O-CH₃ steht, wenn zwischen den Atomen 22 und 23 eine Einfachbindung vorliegt; und R₄ für HO- oder HO-N= steht, in freier Form oder in Form eines physiologisch verträglichen Salzes.

## Beschreibung

Die vorliegende Erfindung betrifft veterinärmedizinische Präparate, die eine Kombination von 1-[4-Chlor-3-(3-chlor-5-trifluormethyl-2-pyridyloxy)phenyl]-3-(2,6- difluorbenzoyl)-harnstoff [nachfolgend Verbindung ( B )] und einem weiteren Wirkstoff der nachstehenden Formel (i) aus der Avermectinklasse enthalten. Sie betrifft ferner die Verwendung dieser beiden Komponenten zur Herstellung veterinärmedizinischer Präparate und deren gemeinsamen Einsatz in einem Verfahren zur Bekämpfung von Ekto- und Endoparasiten bei Nutz-, Haus- und Schosstieren.

1-[4-Chlor-3-(3-chlor-5-trifluormethyl-2-pyridyloxy)phenyl]-3-(2,6- difluorbenzoyl)harnstoff [= Verbindung ( B )] ist aus der Europäischen Offenlegungsschrift EP-0,079,311 einschliesslich seiner Herstellung bekannt.

Bei dem Wirkstoff aus der Avermectinklasse handelt es sich im Rahmen der vorliegenden Erfindung um eine macrozyklische Verbindung der Formel (i) 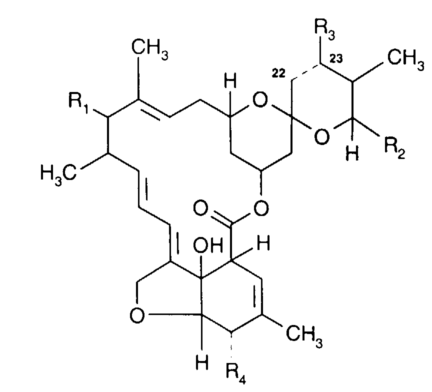 worin R₁ für Wasserstoff oder für einen der Reste 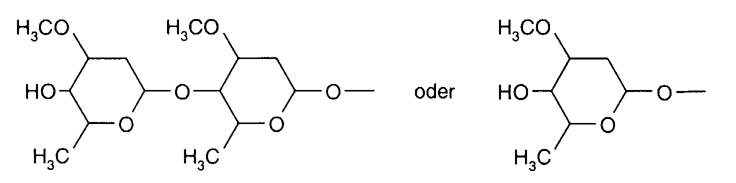 steht; R₂ für -CH(CH₃)-CH₃, -CH(CH₃)-C₂H₅, -C(CH₃)=CH-CH(CH₃)₂ oder Cyclohexyl steht; R₃ für Wasserstoff oder Hydroxy steht, wenn die Bindung zwischen den Atomen 22 und 23 eine Doppelbindung repräsentiert, oder für Wasserstoff oder die Gruppe =N-O-CH₃ steht, wenn zwischen den Atomen 22 und 23 eine Einfachbindung vorliegt; und R₄ für HO- oder HO-N= steht, in freier Form oder in Form eines physiologisch verträglichen Salzes.

Typische Vertreter von Verbindungen der Formel ( i ) sind:
1) Ivermectin ist ein Gemisch zweier Verbindungen der Formel ( i ), worin R₁ für den Rest 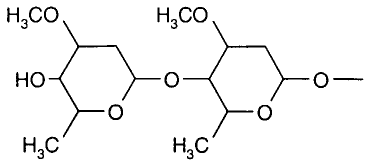 und R₃ für Wasserstoff steht, wobei die Atome 22 und 23 mit einer Einfachbindung verknüpft sind und R₂ entweder für -CH(CH₃)-CH₃ oder -CH(CH₃)-C₂H₅ steht; beide sind einschliesslich ihrer Herstellung aus EP-0,001,689 bekannt. Ivermectin ist im Rahmen der vorliegenden Erfindung bevorzugt.
2) Doramectin ist eine Verbindung der Formel (i). worin R₁ für den Rest 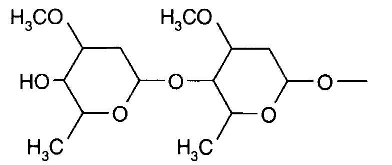 und R₃ für Wasserstoff steht, wobei die Atome 22 und 23 mit einer Doppelbindung verknüpft sind und R₂ für Cyclohexyl steht. Doramectin wird z.B in EP-0,214,731 und EP-0,276,131 beschrieben.
3) Moxidectin, auch bekannt als LL-F28249α ist eine Verbindung der Formel (i), worin R₁ und R₃ für Wasserstoff stehen, wobei die Atome 22 und 23 mit einer Einfachbindung verknüpft sind und R₂ -C(CH₃)=CH-CH(CH₃)₂ bedeutet. Moxidectin ist aus US-4,916,154 bekannt.
4) Selamectin ist 25-Cyclohexyl-25-de(1-methylpropyl)-5-deoxy-22,23-dihydro-5-(hydroxyimino)avermectin B1 monosacharid und somit eine Verbindung der Formel (i), worin R₁ für den Rest 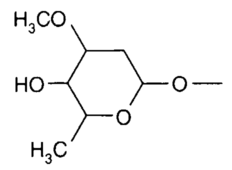 R₂ cyclohexyl bedeutet, R₃ für Wasserstoff steht, wobei die Atome 22 und 23 mit einer Einfachbindung verknüpft sind und R₄ für HO-N= steht. Selamectin ist z.B bekannt aus: ECTOPARASITE ACTIVITY OF SELAMECTIN; A novel endectocide for dogs and cats. A Pfizer Symposium, held in conjunction with The 17th international Conference of the World Association for the Advancement of Veterinary Parasitology, 19 August 1999 . Copenhagen, Denmark.

Die Verbindungen der Formel (i) sind aus den genannten Druckschriften bekannt oder analog zu den bekannten Vertretern erhältlich.

Die Lebenszyklen der verschiedenen Parasiten, die Menschen oder Tiere befallen können, sind bekanntlich sehr komplex, was ihre Bekämpfung in vielen Fällen erschwert. Ausserdem stellen Mehrfacherkrankungen oft ein noch unzureichend gelöstes Problem dar. Vor allem in wärmeren Regionen, werden z.B. Herdentiere, wie Kühe und Schafe, oft epidemisch von Helminthen und anderen Wurmerkrankungen befallen. Diese Erkrankungen sind nicht selten lebensbedrohlich. Vielfach werden diese bereits durch die Endoparasiten geschwächten Tiere auch noch zusätzlich von Ektoparasiten, vor allem Zecken, heimgesucht, die nicht selten in Massen auftreten und diese Tiere noch mehr schwächen und einen zusätzlichen Stressfaktor bilden. Die an sich schon durch die Würmer geschwächten Tiere nehmen wegen dieser Plagegeister noch weniger Nahrung auf, verlieren noch rascher an Gewicht und benötigen eine intensive Behandlung und Zuwendung. Zecken können überdies mit den unterschiedlichsten Krankheitserregern infiziert sein und diese besonders leicht an bereits geschwächte Wirtstiere übertragen deren Immunsystem ohnehin schon geschwächt ist. Es besteht daher ein vitales Bedürfniss an Präparaten, die sowohl die parasitären Würmer als auch Zecken erfolgreich beseitigen.

Typische Wurmerkrankungen im Rahmen der vorliegenden Erfindung sind solche, die durch Vertreter aus der Klasse Nematoda verursacht werden. Hierzu gehören beispielsweise die Familien Filariidae und Setariidae, und die Gattungen Haemonchus, Trichostrongylus, Ostertagia, Nematodirus, Cooperia, Ascaris, Bunostumum, Oesophagostonum, Chabertia, Trichuris, insbesondere Trichuris vulpis, Strongylus, Trichonema, Dictyocaulus, Capillaria, Strongyloides, Heterakis, Toxocara, insbesondere Toxocara canis, Ascaridia, Oxyuris, Ancylostoma, insbesondere Ancylostoma caninum, Uncinaria, Toxascaris und Parascaris; Dirofilaria, besonders Dirofilaria immitis (Herzwurm).

Zecken können sich ausschliesslich von einem oder auch vom Blut verschiedener Wirte ernähren. Sie setzen sich auf dem Wirtstier fest und saugen dessen Blut. Die mit Blut vollgesaugten Weibchen fallen vom Wirtstier ab und legen dann eine grosse Zahl von Eiern in einer geeigneten Nische in der Umgebung. Die sich entwickelnden Larven suchen hierauf ein neues Wirtstier, um sich via das Nymphenstadium zu Adulten zu entwickeln und sich wiederum mit Blut vollzusaugen. Gewisse Arten können von einem zu einem zweiten und sogar zu einem dritten Wirtstier weiterwandern.

Zecken, welche hier von Bedeutung sind, gehören vor allem den Gattungen Amblyomma, Boophilus, Hyalomma, Ixodes, Rhipicephalus und Dermacentor an, besonders handelt es sich jedoch um die Arten Boophilus microplus und B. annulatus, ganz besonders B. microplus. Sie sind verantwortlich für die Übertragung zahlreicher Krankheiten welche Mensch und Tier befallen können. Vor allem werden bakterielle, protozoäre, rickettsiale und virale Krankheiten übertragen. Die Erreger solcher Krankheiten werden insbesondere von Zecken, welche sich von mehr als einem Wirt ernähren, übertragen. Diese Krankheiten können zur Schwächung oder gar zum Tod der Wirtstiere führen. Sie verursachen meistens einen grossen ökonomischen Schaden, indem beipielsweise das Fleisch der Nutztiere an Wert verliert, die verwendbare Haut beschädigt oder die Milchproduktion verringert wird.

Zecken der oben aufgeführten Arten werden traditionell bei ansonsten gesunden Wirtstieren durch Behandlung der befallenen Tiere mit einem akarizid wirksamen Mittel entsprechend dem aktuellen Befall, also kurativ, bekämpft. Das Auftreten der Zecken, beispielsweise auf der Weide, hängt jedoch stark von den saisonalen Wetterbedingungen und der schlussendliche Befall der Wirtstiere zudem auch von deren Resistenz gegen die Zecken ab. Dies bedeutet, dass eine präventive Bekämpfung der Zecken schwierig und zeitraubend ist, da u.a. Befallsdruck durch die Schädlinge nur schwer abzuschätzen ist. Bei Tieren die bereits durch andere Parasiten, wie z.B. durch die genannten Würmer, geschwächt sind, ist eine wirkungsvolle Kontrolle der Zecken besonders wichtig. Hier sind Präparate bevorzugt, die eine ausgeprägte Kontaktwirkung zeigen, d.h. die Zecke entweder bereits beim Kontakt mit dem Fell oder der Haut abtöten oder sie veranlassen garnicht erst sich festzusaugen. Dabei ist es bei geschwächten Tieren besonders wichtig, dass man sie durch die Antizeckenbehandlung nicht zusätzlich stresst und sie nicht mit einer Vielzahl veterinärmedizinischer Präparate behandelt, deren unerwünschte Nebenwirkungen sich akkumulieren könnten. Massgeschneiderte, gut verträgliche Breitbandpräparate könnten hier Abhilfe schaffen.

Es wird jedoch zunehmend schwieriger neue Wirkstoffklassen zu synthetisieren oder aus natürlichen Quellen zu isolieren, die den bereits verfügbaren Wirksubstanzen ebenbürdig oder diesen sogar überlegen sind. Viele der bekannten Wirstoffe auf dem Gebiet der Tiergesundheit zeigen eine ausserordentlich ausgeprägte Aktivität gegen bestimmte Zielparasiten. Leider beschränkt sich ihre Wirkung meistens entweder nur auf äussere oder nur auf innere Parasiten oder sie zeigen erhebliche Lücken in ihrem Wirkungsspektrum in der einen oder anderen Richtung. Bei der Behandlung von bereits geschwächten Tieren wäre es jedoch wünschenswert, wenn Breitbandpräparate zur Verfügung stünden, die ein breites Wirkungsspektrum abdecken, sehr gut verträglich sind und die Zahl der Behandlungen auf ein Minimum reduzieren können.

Statt neue Wirkstoffe, möglicherweise jahrelang erfolglos zu suchen, kann auch der Versuch unternommen werden, die gewünschte Breitbandwirkung durch Kombination von bekannten Wirksubstanzen zu erzielen. Dies schein im ersten Moment eine einfache Aufgabe zu sein, da die Wirkungsspektren verschiedener Stoffklassen hinlänglich bekannt sind. In Wirklichkeit führt die blosse Kombination zweier Wirksubstanzen aber nur selten zu dem gewünschten Erfolg, da die gleichzeitige Verabreichung unterschiedlicher Wirksubstanzen zu nicht vorhersagbaren kinetischen und metabolischen Effekten führen kann, ganz zu schweigen von Potenzierungen bei den unerwünschten Nebeneffekten. Auch gegenseitiger Verstärkung oder Abschwächung werden beobachtet und sogar unerwünschte chemische Reaktionen zwischen durch die körpereigenen Enzyme entstehenden Abbauprodukte. Nicht alle, der vorgeschlagenen Kombinationen zeigen beim praktischen Einsatz das gewünschte Breitbandspektrum. In manchen Fällen treten neue Wirkungslücken auf, die das Präparat für den geplanten Einsatz wertlos machen oder ihn auf Sonderfälle beschränken, da der eine oder andere häufig auftretende Parasit nicht oder nur ungenügend erfasst oder das Immunsystem der behandelten Tiere negativ beeinflusst wird, und sie werden z.B. für Pilzerkrankungen oder andere Sekundärinfektionen anfällig, was ihre Behandlung zusätzlich erschwert und den Einsatz weiterer veterinärmedizinischer Präparate nach sich zieht. Als weitere Schwiergkeit kommt hinzu, dass Wurmerkrankungen sich besonders effektiv bekämpfen lassen, wenn man die entsprechenden Präparate systemisch, d.h. entweder perkutan oder oral verabreicht und sie über die Blutbahn den Parasiten erreichen. Zeckenmittel werden dagegen vorzugsweise topikal, d.h. auf die Haut bzw. auf das Fell des Wirtstieres aufgetragen und entfalten dort durch Kontakt ihre Antizeckenwirkung. Es ist ein schwieriges Unterfangen Wirkstoffe zu finden, die in beide Wirkungsrichtungen wirken sollen und sich entweder systemisch oder topical oder gar auf beiden Wegen applizieren lassen.

Will man z.B. Wurmerkrankungen und Zeckenbefall gleichzeitig erfassen, so findet man in der Literatur bereits Vorschläge für Kombinationspräparate. So wird z.B in der Europäischen Offenlegungschrift EP-0,242,502 die Kombination von Avermectinen mit bestimmten Benzoylharnstoffen zur gleichzeitigen Bekämpfung von Insekten, Akarina und Nematoden allerdings im Bereich des Pflanzenschutzes vorgeschlagen.

Versucht man nun diese Erfahrungen aus dem Pflanzenschutz für die Anwendung im veterinärmedizinischen Bereich zu übertragen, so stellt man rasch fest, dass das Wirkungsspektrum der vorgeschlagenen Kombinationen hinsichtlich der Ektoparasiten deutliche Lücken aufweist. Zwar führt der Avermectinanteil zu einer sehr guten Wirkung gegen verschiedene Nematoden, aber der Zusatz der vorgeschlagenen Benzoylharnstoffe zeigt nicht die gewünschte Aktivität im Bereich der Ektoparasiten, insbesondere gegen die wichtigsten Vertreter aus der Ordnung Akarina (Milben und vor allem Zecken).

Jetzt wurde überraschen festgestellt, dass eine relative geringfügige strukturelle Modifikation bei der in EP-0,242,502 vorgeschlagenen Benzoylharnstoffkomponente nicht nur dieses Aktivitätsdefizit ausgleicht, sondern zu äusserst gut verträglichen Präparaten führt, die rasch und anhaltend gegen verschiedene Wurmerkrankungen und gegen Zecken und Milben wirken und auf diese Weise sich für den Einsatz im Bereich der Tiergesundheit hervorragend eignet. Die Verabreichung an Kühe und Schafe zeigt ausserdem, dass diese neuen Kombinationspräoarate weder das normale Verhalten der Tiere untereinander noch deren Fressgewohnheiten nachteilig beeinflusst. Dabei lassen sich die neuen Kombinationen nicht nur kurativ, sondern auch präventiv und auch über längere Zeitphasen mit erhöhtem Befallsrisiko anwenden, ohne, dass negative Nebenefekte in Erscheinung treten und die Tiere belasten.

Die in EP-0,242,502 vorgeschlagenen Benzoylharnstoffe zeichnen sich strukturell durch eine para-Phenoxy- oder para-Pyridyloxyphenylgruppe aus. Ein hervorgehobener Benzoylharnstoff ist z.B. die nachfolgende Substanz ( A ) [in EP-0,242,502 = Substanz llb, Seite 3]:

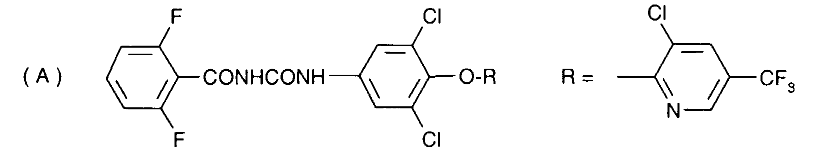

Kombiniert man nun diese Substanz ( A ) mit typischen Vertretern von Avermectinderivaten, so stellt man bei der Kombination mit Ivermectin, Doramectin und Moxidectin fest, dass diese sich für den Einsatz in der Tiergesundheit nicht eignen, da sie zwar die parasitären Würmer rasch und nachhaltig beseitigen, aber offensichtlich keinen Einfluss auf Zecken zeigen.
Vergleicht man die biologische Aktivität dieser im Stand der Technik vorgezeichneten Kombinationen mit derjenigen der nunmehr vorgeschlagenen Kombination, die als ersten Mischungspartner ebenfalls Ivermectin, Doramectin oder Moxidectinund als zweiten Partner den nachfolgenden Benzoylharnstoff ( B ): 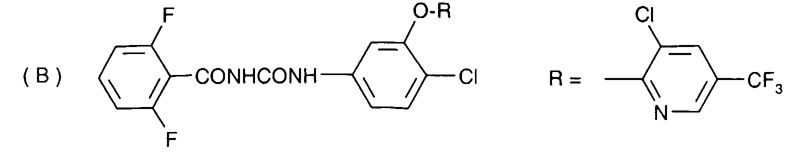 enthalten, so stellt man fest, dass man durch die Verwendung dieses Strukturisomeren ( B ), bei dem ein auch noch ein Chloratom fehlt, man zu einem signifikant breiteren und in der Veterinärmedizin wichtigen Wirkungsspektrum gelangt, das jetzt neben häufig auftretenden Würmern auch Milben und vor allem Zecken umfasst und bei Verabreichung in gegen die Zielparasiten wirksamen Dosierungen beim Wirtstier zu keinerlei unerwünschten Nebeneffekte bewirkt. Ausserdem stellt man überraschend fest, dass die erfindungsgemässen Kombinationen sich systemisch und topikal verabreichen lassen.

Die folgende Beispiele dienen zur weiteren Erläuterung der Erfindung; sollen diese jedoch nicht einschränken.

### Biologisches Beispiel:

Wegen der einfacheren Handhabbarkeit wird die Hühnermilbe *Dermanyssus gallinae,* die ein gutes Modell für die Ermittlung einer Wirkung gegen Zecken darstellt, eingesetzt.

### In vitro Wirkung gegen Dermanyssus gallinae

Der Versuch wird mit Verdünnungsreihen durchgeführt [Avermectinkomponente in Konzentrationen von (32), 10, 3.2, 1.0, 0.32 bis 0.0001 ppm; den 1-[4-Chlor-3-(3-chlor-5-trifluormethyl-2-pyridyloxy)phenyl]-3-(2,6- difluorbenzoyl)harnstoff in Konzentrationen von 100, 32, 10, 3.2, 1.0, 0.32 und 0.1 ppm] durchgeführt. Für den Test wird jeweils die niedrigste Konzentration der einen Wirksubstanz mit der niedrigsten Konzentration der anderen Wirksubstanz kombiniert und so die Grenzen der Aktivität ausgelotet. 10 auf eine Klebefolie aus Kunststoff aufgeklebte, vollgesaugte weibliche Milben der Gattung *Dermanyssus gallinae* werden mit 50 µl einer wässrigen Suspension bzw. Emulsion der jeweiligenTestkombination in Kontakt gebracht. Nach Trocknung wird die Folie auf eine Glasscheibe geklebt. Auf diese Weise befindet sich jede Milbe in einer Art Luftblase, deren Unterseite von der Glasscheibe und deren Oberseite durch eine Ausstülpung der Klebefolie gebildet wird. Diese Blase enthält genügend Luft, damit die Milbe nicht erstickt. Nach 5 Tagen wird die Wirkung der Testsubstanz mit Hilfe eines Stereomikroskops ausgewertet, indem man die Auswirkung auf die Mortalität, Eiablage, Eiqualität, Schlupfrate, Verpup-pungsrate und Entwicklung von Protonymphen nach folgenden 4 Gesichtspunkten auswertet:
1) wenn 9 bis 10 Milben tot sind, liegt eine lethale Wirkung vor (gekennzeichnet mit M);
2) falls 2 oder mehrere Milben überleben, aber keine Eier produzieren, liegt Sterilität (S) vor;
3) falls 2 oder mehrere Milben überleben und Eier produzieren, aus denen aber keine Larven schlüpfen und sich auch keine Protonymphen entwickeln, liegt eine entwicklungshem-mende Wirkung (H) vor;
4) falls 2 oder mehrere Milben überleben und die übliche Anzahl von normalen Eiern legen, aus denen Larven schlüpfen, die sich zu Protonymphen weiterentwickeln, liegt keine Aktivität vor (-).

Bei jedem Versuch auch zwei leere Suspensionen/Emulsionen (ohne Wirkstoff) und eine Suspension/Emulsion mit jeweils nur einer Komponente in den entsprechenden Konzentrationen mit getestet.

Der Versuch wird mit den nachfolgend genannten Kombinationen dreimal wiederholt und die Resultate gemittelt. Das Endresultat ist in den Tabellen 1 bis 3 aufgelistet, wobei in den mit a gekennzeichneten Tabellen die Kombinationen aus dem Stand der Technik und bei den mit b gekennzeichneten Tabellen die Ergebnisse der erfindungsgemässen Kombinationen aufgeführt werden.

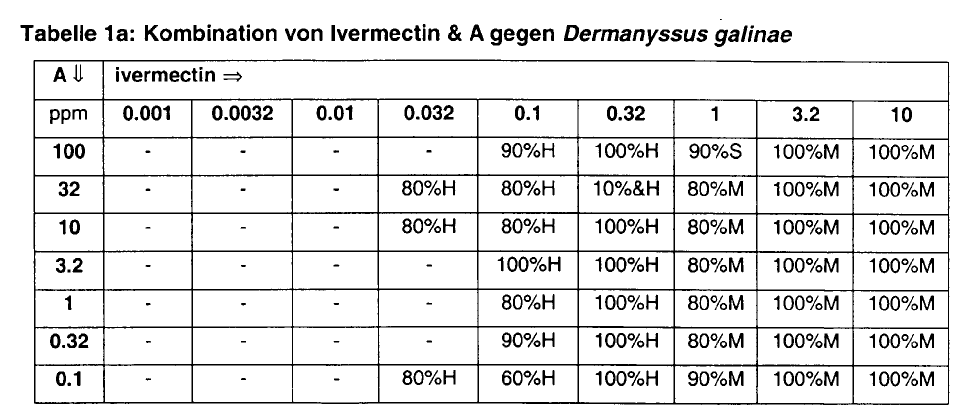

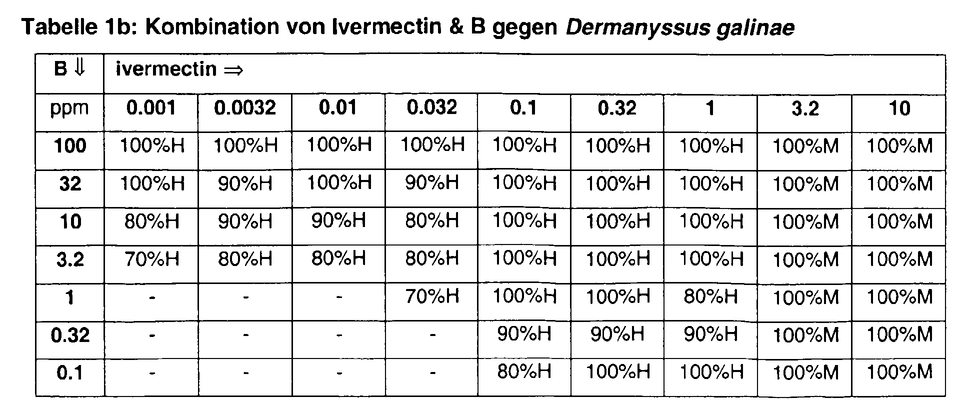

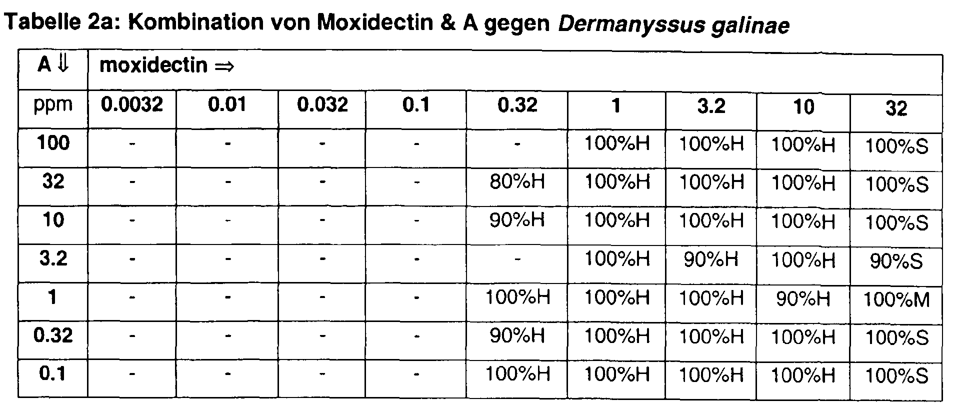

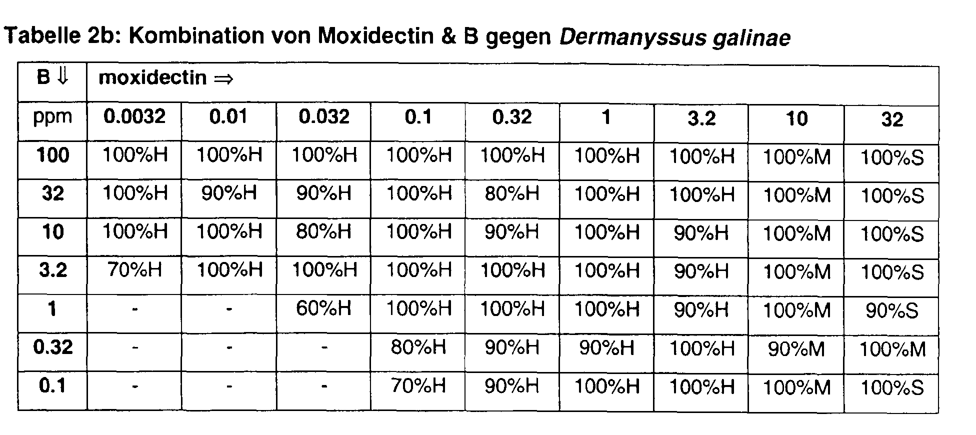

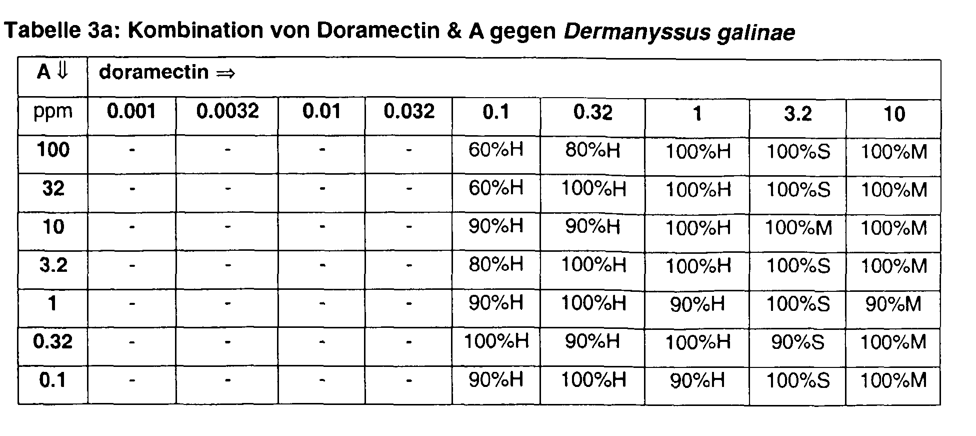

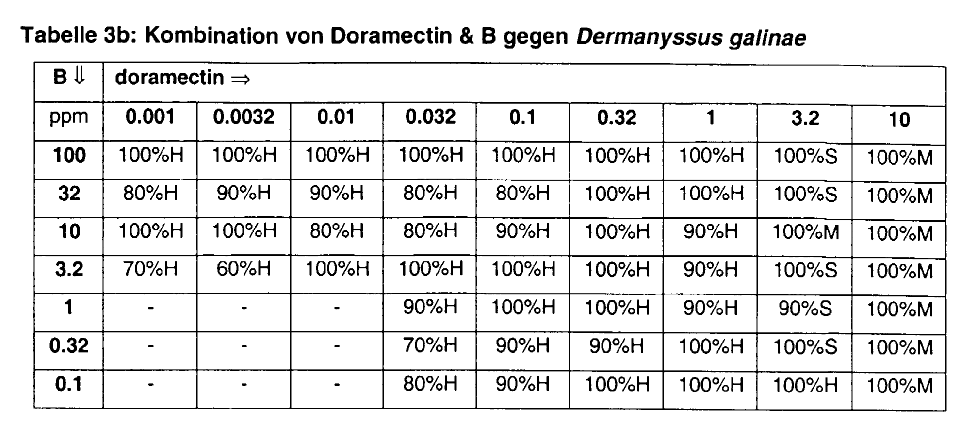

Alle leeren Suspensionen/Emulsionen (ohne Wirkstoff) zeigen keine biologische Aktivität, aber auch keine Nebenreaktionen. Die Wirksubstanz ( A ) des Standes der Technik zeigt bein keiner Konzentration eine Aktivität; die Avermectinkomponenten Ivermectin, Moxidectin und Doramectin erzielen bei 0.32 und 10 ppm volle Wirkung im Sinne der Verhinderung einer Eiablage. Der 1-[4-Chlor-3-(3-chlor-5-trifluormethyl-2-pyridyloxy)phenyl]-3-(2,6-difluorbenzoyl)harnstoff verhindert die Eiablage bis zu einer Konzentration von 0.01 ppm.

Der Vergleich zeigt anschaulich, dass die erfindungsgemässen Kombinationen (vgl. Tabellen 1b, 2b und 3b mit 1a, 2a und 3a) bis zu wesentlich niedrigeren Grenzkonzentrationen gegen *Dermanyssus gallinae* aktiv sind, als die entsprechende Vergleichs-kombination aus dem Stand der Technik. Einzelversuche mit der Zecke *Boophilus microplus* zeigen ein völlig analoges Erscheinungsbild und erste *in* vivo-Versuche an Schafen bestätigen die angestrebte Aktivität. *In vitro* Versuche gegen Nematoden *(Haemonchus contortus)* zeigen, das die Zumischung der Substanz ( B ) zu der Avermectinkomponente keinen Einfluss auf deren Antiwurmwirkung hat.

Es wurde auch festgestellt, dass man mit Hilfe bestimmter Applikationsarten, beispielsweise durch äussere Anwendung, insbesondere aber durch systemische Applikation erfindungsgemässen Kombinationen und gegebenenfalls zur Verstärkung deren Wirkung unter Zusatz einer oder mehrerer Verbindungen ausgewählt aus den Verbindungen aus anderen Substanzklassen, wie z.B. Methoprene, Hydroprene, Dicyclanil und Cythioate oder deren Salze, die erwähnten Ektoparasiten sehr rasch und vollständig beseitigen kann, dadurch dass man blockierend in ihren komplexen Entwicklungszyklus eingreift, und gleichzeitig eine effiziente Bekämpfung von Endoparasiten, insbesondere Nematoden, erreicht. Die erfindungsgemässen Kombinationen und Präparate entfalten ihre ausgezeichnete parasitizide Wirkung auch dann noch, wenn man sie systemisch, d.h. oral, parenteral, subkutan, intramuskulär oder intravenös an das Wirtstier verabreicht. Dadurch ist es ist jetzt möglich, durch ihre gezielte periodische Verabreichung ständig wiederkehrende Reinfestationen der Wirtstiere mit den verschiedenen Parasiten auf einfache Weise zu verhindern und die Parasiten dauerhaft von den Herden fernzuhalten. Die Parasiten werden dabei entweder durch Konatakt getötet oder an ihrer Vermehrung gehindert, oder die juvenilen Stadien werden am Heranwachsen gehindert und sterben frühzeitig ab.

Ein weiterer bevorzugter Gegenstand der vorliegenden Erfindung ist besteht somit auch in einem Verfahren zur gleichzeitigen Bekämpfung von Nematoden, Milben und Zecken in und an Haus-, Nutz- und Schosstieren, dadurch gekennzeichnet, dass man die erfindungsgemässe Wirkstofkombination oder ein veterinermedizinisches Präparat, das die Kombination enthält, oral, parenteral oder durch Implantat in einer parasitizid wirksamen Menge an das Wirtstier verabreicht.

Erfindungswesentlich ist, dass die erfindungsgemässe Kombination bzw. das Präparat so verabreicht wird, dass die Wirksubstanzen, welche das Mittel enthält, von Endoparasiten, Ektoparasiten sowie auch von weiteren Schädlingen, welche als Vektoren für die Übertragung von Endoparasiten in Frage kommen, mit dem Blut des Wirtstieres in ausreichender Menge aufgenommen werden können, so dass die von den adulten Schädlingen abgelegten Eier beziehungsweise die daraus schlüpfenden Larven sich nicht mehr weiterentwickeln.

Dies wird mit dem erfindungsgemässen Kombinationen bzw. Präparaten durch Anwendung verschiedener Applikationsformen erreicht, z.B. indem man das die Wirkstoffe enthaltende Präparate oral verabreicht. Formuliert heisst in diesem Fall z.B. in Form eines Pulvers, einer Tablette, eines Granulats, einer Kapsel, einer Emulsion, eines Schaumes, im mikro-enkapsulierter Form, etc., wobei man das Präparat dem Tier nicht unbedingt direkt zu geben braucht, sondern z.B. unter das Futter mischt. Selbstverständlich können alle oral zu verabreichenden Kompositionen neben üblichen Formulierungsstoffen weitere Zusätze enthalten, die die freiwillige Aufnahme durch das Wirtstier fördern, z.B. geeignete Duft- und Aromastoffe. Die orale Verwendung ist, auf Grund ihrer einfachen Ausführbarkeit, eine der bevorzugten Gegenstände dieser Erfindung. Eine weitere Applikationsart ist der parenterale Einsatz, z.B. durch subkutane oder intravenöse Injektion, topikale Applikation oder als Langzeitpräparat (Depotform) in Form eines Implantates oder Injektion von Mikrokapseln (sog. "Mikrosphären").

Die orale Applikation schliesst z.B. das Verabreichen von Tierfutter, beispielsweise von Hunde- oder Katzenfutter ein, das die Aktivsubstanzen bereits beigemischt enthält, z.B. als Biskuits, als Kautablette, als wasserlösliche Kapseln oder Tabletten, in wasserlöslicher Form, die auf das Futter getropft werden kann oder in sonstigen, dem Tierfutter beimeng-baren Formen. Die Implantate schliessen auch alle Vorrichtungen ein, die in den Körper des Tieres zur Substanzabgabe eingebracht werden können.

Perkutane Applikationsformen schliessen beispielsweise die subkutane, dermale, intramuskuläre und sogar intravenöse Verabreichung injizierbarer Formen ein. Dabei können ausser den üblichen Injektionsspritzen mit Nadeln auch nadellose Druckstossapparate, aber auch Pour-on- und Spot-on-Formulierungen zweckdienlich sein.

Durch Wahl einer geeigneten Formulierung ist es möglich das Eindringungsvermögen der Wirkstoffe durch das lebende Gewebe des Tieres zu fördern bzw. Ihre Verfügbarkeit aufrechtzuerhalten. Dies ist von Bedeutung, wenn z.B. ein oder mehrere schwerlösliche Wirkstoffe eingesetzt werden, deren geringe Löslichkeit eine löslichkeitsfördernde Massnahme erfordern, da die Körperflüssigkeit des Tieres nur geringe Mengen der Wirkstoffe auf einmal zu lösen vermag.

Ferner können die Wirkstoffkombinationen auch in einer Matrixformulierung vorliegen, die auf physikalische Weise ihre Zersetzung verhindert und die laufende Verfügbarkeit der Wirkstoffe aufrechterhält. Diese Matrixformulierung wird in den Körper injiziert und verbleibt dort als eine Art Depot, aus dem die Wirkstoffkombination kontinuierlich freigesetzt wird. Derartige Matrixformulierungen sind dem Fachmann bekannt. Es handelt sich im allgemeinen um wachsartige, halbfeste Excipientien, wie z.B. pflanzliche Wachse und Polyethylenglykole mit hohem Molekulargewicht oder Copolymere aus abbaubarern Polyestern.

Eine hohe Verfügbarkeit der Wirkstoffkombination wird auch durch Einführung eines Implantats der Aktivsubstanzen in das Tier erhalten. Solche Implantate sind in der Veterinärmedizin weit verbreitet und bestehen oftmals aus silikonhaltigem Gummi. Die Aktivsubstanzen sind dabei im festen Gummi dispergiert oder befinden sich im Innern eines Gummihohlkörpers. Zu beachten ist dabei, dass Aktivsubstanzen ausgewählt werden, die im Gummiimplantat löslich sind, da sie zuerst im Gummi gelöst werden und dann kontinuierlich aus dem Gummimaterial in die Körperflüssigkeit des zu behandelnden Tieres sickern.

Die Freigaberate der Aktivsubstanzen aus dem Implantat und damit die Zeitspanne, in der das Implantat eine Wirkung zeigt, wird im allgemeinen von der Genauigkeit der Eichung (Wirkstoffmenge im Implantat) des Implantats, der Umgebung des Implantats und der Polymerformulierung aus der das Implantat hergestellt ist, bestimmt.

Die Verabreichung der Wirkstoffe mittels Implantat stellt einen weiteren bevorzugten Bestandteil der vorliegenden Erfindung dar. Eine derartige Verabreichung ist äusserst ökonoisch und effektiv, weil ein richtig dimensioniertes Implantat eine konstante Konzentration der Wirksubstanzen im Gewebe des Wirtstieres gewährleistet. Implantate können heutzutage derart gestaltet und in einfacher Weise implantiert werden, dass sie die Wirkstoffe über einige Monate zu liefern im Stande sind.

Die Verabreichung von veterinärmedizinischen Zusätzen zum Tierfutter ist auf dem Gebiet der Tiergesundheit bestens bekannt. Ueblicherweise wird zunächst ein sogenanntes Premix (Vormixtur) hergestellt, in dem die Aktivsubstanzen in einer Flüssigkeit dispergiert oder feinerteilt in festen Trägermaterialien vorliegt. Dieses Premix kann normalerweise, in Abhängigeit von der gewünschten Endkonzentration im Futter, etwa 1 bis 800 g der Wirkstoffkombination pro kg Premix enthalten.

Es ist zudem bekannt, dass die Wirkstoffe durch die Bestandteile des Futters unter ungünstigen Umständen hydrolysiert oder geschwächt werden können. Dies kann durch Einarbeitung in eine Schutzmatrix, z.B. in Gelatine,verhindert werden.

Die erfindungsgemässen Kombinationen werden zweckmässigerweise in einer Dosierung von 0,01 bis 800, vorzugsweise 0,1 bis 200, insbesondere 0,5 bis 50 mg/kg Körpergewicht in Bezug auf das Wirtstier appliziert, wobei die Mengenangaben sich auf die Summe beider Aktivsubstanzen beziehen.

Eine gute Dosis, die regelmässig an das Wirtstier verabreicht werden kann, liegt insbesonere bei der Katze bei 2,5 - 5 mg/kg Körpergewicht erfindungsgemässen Kombinationen und beim Hund bei 0,5 - 15 mg/kg pro kg Körpergewicht. Beim Schaf bei 0,5 - 30 mg/kg Körpergewicht und bei der Kuh bei 1 - 30 mg/kg Körpergewicht. Die Verabreichung erfolgt zweckmässigereise in regelmässigen Zeitabständen, z.B. nach einigen Tagen, wöchentlich oder monatlich.

Die Gesamtdosis kann von einer Tiergattung zur anderen und auch innerhalb einer Tiergattung variieren, da sie u.a. vom Gewicht und der Konstitution des Tieres abhängt.

Als Formulierungshilfsstoffe für Mittel zum Einsatz bei Menschen, Haus-, Nutz- und Schossieren können die aus der veterinärmedizinischen Praxis für orale, parenterale und Implanate bekannten Materialien eingesetzt werden. Nachfolgend seien einige nicht-limitierenden Beispiele genannt.

Die erfindungsgemässe Wirkstoffkombinationen enthalten 1-[4-Chlor-3-(3-chlor-5-trifluor-methyl-2-pyridyloxy)phenyl]-3-(2,6- difluorbenzoyl)harnstoff [Verbindung B] und eine Verbindung der Formel (i) [Verbindung (i)] in nahezu jedem Werhältnis zueinander, vorzugsweise jedoch in einem Mischungsverhältnis von B : 1 von 100'000 : 1 bis 1 : 100, insbesondere in einem Verhältnis zwischen 10 : 1 bis 1: 100, wobe vorteilhafte Wirkstoffkonzentrationen bei etwa 100 -1 ppm von B bis 0,1 - 10 ppm von (i) liegen.

Erfindungsgemäss anzuwendende Mittel bzw Präparate enthalten in der Regel 0,1 bis 99 Gew.-%, insbesondere 0,1 bis 95 Gew.-%, einer Kombination der Verbindung 1-[4-Chlor-3-(3-chlor-5-trifluormethyl-2-pyridyloxy)phenyl]-3-(2,6- difluorbenzoyl)harnstoff und einer Verbindung der Formel (i) und 99,9 bis 1 Gew.-%, insbesondere 99,9 bis 5 Gew.-%, eines festen oder flüssigen, physiologisch verträglichen Trägerstoffs, darunter 0 bis 25 Gew.-%, insbesondere 0,1 bis 25 Gew.-%, eines untoxischen Tensides.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphoshat oder Calciumhydrogenphosphat, ferner Bindemittel, wie Stärkekleister unter Verwenung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxy-ethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieseläure, Talk, Stearinsäure oder Salze davon, wie Magnesium- oder Calciumstearat, und/oder Polyethylenglykol. Dragee-Kerne können mit geeigneten, gegebenenfalls magensaftresisenten, Ueberzügen versehen werden, wobei man u.a. konzentrierte Zuckerlösungen, welche gegebenenfalls arabischen Gummi, Talk, Polyvinylpyrrolidon, Polyethylenglykol und /oder Titandioxid enthalten, oder Lacklösungen in geeigneten organischen Lösungsitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat, verwendet. Den Tabletten- oder Dragee-Ueberzügen können Farbstoffe, Aromastoffe oder Pigmente, z.B. zur Identifizierung oder zur Kennzeichnung verschiedener Dosierungen, beigefügt werden.

Weitere oral anwendbare Präparate sind Steckkapseln aus Gelatine, sowie weiche, geschlossene Kapseln aus Gelatine und einem Weichmacher, wie Glycerin oder Sorbit. Die Steckkapseln können die Wirkstoffe in Form eines Granulats, z.B. im Gemisch mit Füllstofen, wie Lactose, Bindemitteln, wie Stärken und/oder Gleitmitteln, wie Talk oder Magneiumtearat, und gegebenenfalls von Stabilisatoren, enthalten. In weichen Kapseln sind die Wirktoffe vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können. Bevorzugt sind u.a. Kapseln, die sowohl leicht zerbissen als auch unzerkaut gechluckt werden können.

Zur parenteralen Verabreichung eignen sich in erster Linie wässrige Lösungen die Wirkstoffkombination in wasserlöslicher Form, z.B. von wasserlöslichen Salzen, ferner Suspensionen der Wirkstoffe, wie entsprechende ölige Injektionssuspensionen, wobei man geeignete lipophile Lösungsmittel oder Vehikel, wie fette Oele, z.B. Sesamöl, oder synthetische Fettäureester, z.B. Ethyloleat, oder Triglyceride verwendet, oder wässrige Injektionssuspenionen, welche viskositätserhöhende Stoffe, z.B.

Natriumcarboxymethylcellulose, Sorbit und/oder Dextran und gegebenenfalls Stabilisatoren enthalten.

Die erfindungsgemässen Präparaten können in an sich bekannter Weise, z.B. mittels konvenioneller Misch-, Granulier-, Dragier-, Lösungs- oder Lyophilisierungsverfahren hergestellt werden. So kann man veterinärmedizinische Präparate zur oralen Anwendung erhalten, indem man die Wirkstoffkombination mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragee-Kernen verarbeitet.

In den nachfolgenden Formulierungsbeispielen für den Einsatz an Haus-, Nutz- und Schosstieren steht der Begriff "Wirkstoffkombination" stellvertretend für eine 1:1 Kombination von 1-[4-Chlor-3-(3-chlor-5-trifluormethyl-2-pyridyloxy)phenyl]-3-(2,6-difluorbenzoyl)harnstoff und Ivermectin.

**Tabletten:**

| enthaltend einen der Wirkstoffe der Formel I können folgendermassen hergestellt werden: | |
|---|---|
| Bestandteile (für 1000 Tabletten) | |
| Wirkstoffkombination | 25 g |
| Lactose | 100,7 g |
| Weizenstärke | 6,25 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird die Wirkstoffkombination, die Lactose, das Talkum und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben, mit dem Magnesiumstearat vermischt und zu beidseitig konkaven Tabletten von etwa 6 mm Durchmesser verpresst.

**Tabletten:**

| enthaltend total 0,0183 g Wirkstoffkombination werden wie folgt hergestellt: | |
|---|---|
| Zusammensetzung (für 10'000 Tabletten) | |
| Wirkstoffkombination | 183,00 g |
| Lactose | 290,80 g |
| Kartoffelstärke | 274,70 g |
| Stearinsäure | 10,00 g |
| Talk | 217,00 g |
| Magnesiumstearat Kolloidales Siliciumdioxid | 2,50 g |
| | 32,00 g |
| Ethanol | q.s. |

Ein Gemisch der Wirkstoffkombination, der Lactose und 274,70 g Kartoffelstärke wird mit einer ethanolischen Lösung der Stearinsäure befeuchtet und durch ein Sieb granuliert.

Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen werden können.

Kapseln: enthaltend total 0,022 g der Wirkstoffkombination können wie folgt hergestellt werden:

| Zusammensetzung (für 1000 Kapseln) | |
|---|---|
| Wirkstoffkombination | 22,00 g |
| Lactose | 249,80 g |
| Gelatine | 2,00 g |
| Maisstärke | 10,00 g |
| Talk | 15,00 g |
| Wasser | q.s. |

Man mischt die Wirkstoffkombination mit der Lactose, befeuchtet die Mischung gleichmässig mit einer wässrigen Lösung der Gelatine und granuliert sie durch ein Sieb mit einer Maschenweite von 1,2-1,5 mm. Das Granulat mischt man mit der getrockneten Maisstärke und dem Talk und füllt Portionen von 300 mg in Hartgelatinekapseln (Grösse 1) ab.

| Premix (Futterzusatzmittel): | |
|---|---|
| 0,16 | Gewichtsteile der Wirkstoffkombination |
| 4,84 | Gewichtsteile sekundäres Calciumphosphat, Tonerde, Aerosil, Carbonat oder Kalk werden bis zur Homogenität mit |
| 95 | Gewichtsteilen eines Tierfutters gemischt. |
| oder 0,41 | Gewichtsteile der Wirkstoffkombination |
| 5,00 | Gewichtsteile Aerosil/Kalk (1:1) werden bis zur Homogenität mit |
| 94,59 | Gewichtsteilen eines käuflichen Trockenfutters gemischt. |

| Boli: | | |
|---|---|---|
| I | Wirkstoffkombination | 33,00 % |
| | Methylcellulose | 0,80 % |
| | Kieselsäure hochdispers | 0,80 % |
| | Maisstärke | 8,40 % |
| II | Milchzucker krist. | 22,50 % |
| | Maisstärke | 17,00 % |
| | mikrokrist. Cellulose | 16,50 % |
| | Magnesiumstearat | 1,00% |

Zuerst wird die Methylcellulose in Wasser eingerührt. Nachdem das Material gequollen ist, wird Kieselsäure eingerührt und das Gemisch homogen suspendiert. Wirkstoffkombination und Maisstärke werden gemischt. In diese Mischung wird die wässrige Suspension eingearbeitet und zu einem Teig geknetet. Die so erhaltene Masse wird durch ein 12 M-Sieb granuliert und getrocknet. In einem weiteren Schritt werden alle 4 Hilfsstoffe gut gemischt. Zuletzt werden die aus den ersten beiden Teilschritten resultierenden Vormischungen gemischt und zu Boli verpresst.

| Injektabiles | | |
|---|---|---|
| A. Oeliges Vehikel (langsame Freisetzung) | | |
| 1. | Wirkstoffkombination | 0,1-1,0 g |
| | Erdnussöl | ad 100 ml |
| 2. | Wirkstoffkombination | 0,1-1,0 g |
| | Sesamöl | ad 100 ml |

Herstellung: Die Wirkstoffkombination wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 mm sterilfiltriert.

| B. Wassermischbares Lösungsmittel (mittlere Freisetzungsgeschwindigkeit) | |
|---|---|
| Wirkstoffkombination | 0,1-1,0 g |
| 4-Hydroxymethyl-1,3-dioxolan (Glycerol | Formal) 40 g |
| 1,2-Propandiol | ad 100 ml |
| Ein Wirkstoff aus Tabelle 1 | 0,1-1,0 g |
| Glycerindimethylketal | 40 g |
| 1,2-Propandiol | ad 100 ml |

Herstellung: Die Wirkstoffkombination wird in einem Teil des Lösungsmittels unter Rühren gelöst, auf das Sollvolumen aufgefüllt und durch ein geeignetes Membranfilter mit 0.22 mm sterilfiltriert.

| C. Wässeriges Solubilisat (rasche Freisetzung) | | |
|---|---|---|
| 1. | Wirkstoffkombination | 0,1-1,0 g |
| | Polyethoxyliertes Ricinusöl (40 Aethylenoxideinheiten) | 10 g |
| | 1,2-Propandiol | 20 g |
| | Benzylalkohol | 1 g |
| | Aqua ad injekt. | ad 100 ml |
| 2. | Wirkstoffkombination | 0,1-1,0 g |
| | Polyethoxyliertes Sorbitanmonooleat (20 Aethylenoxideinheiten) | 8 g |
| | 4-Hydroxymethyl-1,3-dioxolan (Glycerol Formal) | 20 g |
| | Benzylalkohol | 1 g |
| | Aaua ad iniekt. | ad 100 ml |

Herstellung: Die Wirkstoffkombination wird in den Lösungsmitteln und dem Tensid gelöst und mit Wasser auf das Sollvolumen aufgefüllt. Sterilfiltration durch geeignetes Membranfilter mit 0,22 mm Porendurchmesser.

| Pour-on | | |
|---|---|---|
| A. | Wirkstoffkombination | 5 g |
| | Isopropylmyristat | 10g |
| | Isopropanol | ad 100 mL |
| B. | Wirkstoffkombination | 2 g |
| | Hexyllaurat | 5 g |
| | mittelkettige Triglyceride | 15 g |
| | Ethanol | ad 100 mL |
| C. | Wirkstoffkombination | 2 g |
| | Oleyloleat | 5 g |
| | N-Methyl-pyrrolidon | 40 g |
| | Isopropanol | ad 100 mL |

Die wässerigen Systeme können bevorzugterweise auch für die orale und/oder intraruminale Applikation eingesetzt werden.

Die Mittel können auch weitere Zusätze wie Stabilisatoren, z.B. gegebenenfalls epoxidierte Pflanzenöle (epoxidiertes Kokosnussöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Es können zudem weitere biologisch aktive Substanzen oder Zusätze, die sich gegenüber den Verbindungen der Formel I neutral verhalten und keinen schädlichen Einfluss auf das zu behandelnde Wirtstier haben, sowie Mineralsalze oder Vitamine den beschriebenen Kompositionen zugesetzt werden.

## Patentansprüche

1. Verwendung einer Kombination von 1-[4-Chlor-3-(3-chlor-5-trifluormethyl-2-pyridyloxy)-phenyl]-3-(2,6-difluorbenzoyl)harnstoff und einer Verbindung der Formel (i) 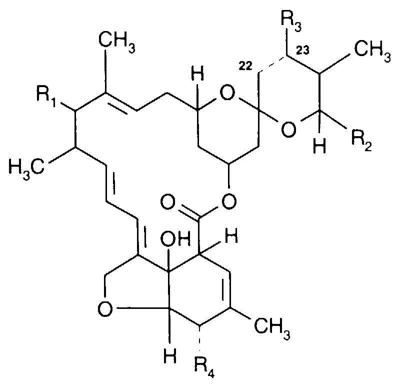 worin R₁ für Wasserstoff oder für einen der Reste 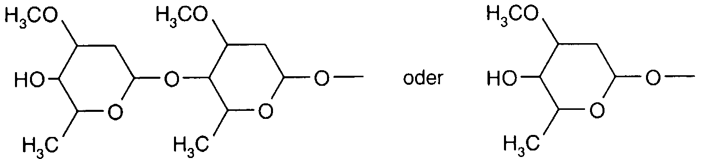 steht; R₂ für -CH(CH₃)-CH₃, -CH(CH₃)-C2H₅, -C(CH₃)=CH-CH(CH₃)₂ oder Cyclohexyl steht; R₃ für Wasserstoff oder Hydroxy steht, wenn die Bindung zwischen den Atomen 22 und 23 eine Doppelbindung repräsentiert, oder für Wasserstoff oder die Gruppe =N-O-CH₃ steht, wenn zwischen den Atomen 22 und 23 eine Einfachbindung vorliegt; und R₄ für HO- oder HO-N= steht, in freier Form oder in Form eines physiologisch verträglichen Salzes in einem Verfahren zur Bekämpfung von Ekto- und Endoparasiten bei Nutz-, Haus- und Schosstieren.

2. Verwendung nach Anspruch 1, dadurch gekennzeichnet, dass es sich bei der Verbindung der Formel (i) um Ivermectin, Doramectin, Moxidectin oder Selanectin handelt.

3. Verwendung nach einem der Ansprüche 1 oder 2, dadurch gekennzeichnet, dass es sich bei den Parasiten um Milben, Zecken und tierparasitäre Nematoden handelt.

4. Verwendung nach einem der Ansprüche 1 oder 2, dadurch gekennzeichnet, dass das Kombinationspräparat zum systemischen Einsatz vorgesehen ist.

5. Verwendung der Wirkstoffkombination nach Anspruch 1 zur Herstellung eines veterinärmedizinischen Kombinationspräparates zur Bekämpfung von Ekto- und Endoparasiten bei Nutz-, Haus- und Schosstieren.

6. Verwendung nach Anspruch 5 zur systemischen Bekämpfung von Ekto- und Endoparasiten bei Nutz-, Haus- und Schosstieren.

7. Verwendung der Wirkstoffkombination nach Anspruch 1 zur Bekämpfung von Ekto- und Endoparasiten bei Nutz-, Haus- und Schosstieren.

8. Verwendung nach Anspruch 7 zur systemischen Bekämpfung von Ekto- und Endoparasiten bei Nutz-, Haus- und Schosstieren.

9. Verwendung nach einem der Ansprüche 1 oder 2, dadurch gekennzeichnet, dass die Wirkstoffkombination 1-[4-Chlor-3-(3-chlor-5-trifluormethyl-2-pyridyloxy)phenyl]-3-(2,6-difluorbenzoyl)harnstoff und eine Verbindung der Formel (i) in einem Mischungsverhältnis von 1:500 bis 500:1 enthält.

10. Wirkstoffkombination zur zur Bekämpfung von Ekto- und Endoparasiten bei Nutz-, Haus- und Schosstieren, dadurch gekennzeichnet, dass sie 1-[4-Chlor-3-(3-chlor-5-trifluor-methyl-2-pyridyloxy)phenyl]-3-(2,6- difluorbenzoyl)harnstoff und eine Verbindung der Formel (i) vorzugsweise in einem Mischungsverhältnis von 100'000 : 1 bis 1 : 100 enthält.
